## Europäisches Patentamt

## European Patent Office

## Office européen des brevets

(19)

(11) Veröffentlichungsnummer: **0 242 326**
**A2**

(12)

# EUROPÄISCHE PATENTANMELDUNG

(21) Anmeldenummer: **87810208.6**

(22) Anmeldetag: **06.04.87**

(51) Int. Cl.4: **C 12 M 3/00**
**C 12 M 1/34**

(30) Priorität: **15.04.86 CH 1496/86**

(43) Veröffentlichungstag der Anmeldung:
**21.10.87 Patentblatt 87/43**

(84) Benannte Vertragsstaaten:
**CH DE FR GB LI NL**

(71) Anmelder: **Pharmatronic AG**
**Kraftwerkstrasse 3**
**CH-4133 Pratteln (CH)**

(72) Erfinder: **Buchmann, Stephan**
**Emil Frey-Strasse 157**
**CH-4142 Münchenstein (CH)**

**Leuenberger, Hans, Prof. Dr.**
**Kreuzackerweg 12**
**CH-4148 Pfeffingen (CH)**

**Reinke, Ciaudia, Dr.**
**Ziegelhofstrasse 19**
**D-7800 Freiburg i.Br. (DE)**

(74) Vertreter: **Zbinden, Paul A. et ai**
**Patentanwaltsbüro Eder AG Münchensteinerstrasse 2**
**CH-4052 Basel (CH)**

(54) **Membran sowie Einrichtung zur Untersuchung der Diffusion einer Substanz, Verfahren zur Herstellung der Membran und Verfahren zum Betrieb der Einrichtung.**

(57) Eine Einrichtung zur Untersuchung der Diffusion einer Substanz weist ein um eine vertikale Achse (11) drehbares, lösbar am Halteteil (17) eines Rotors (13) befestigtes, Donator-Kompartiment (29) auf, das unten durch die Membran (25) gegen ein Akzeptor-Kompartiment (33) abgegrenzt ist. Die Membran (25) weist einen flexiblen Träger aus einem biokompatiblen, leblosen, lichtdurchlässigen Material, auf dem beispielsweise einen Zellrasen bildende Zellen kultiviert wurden. Durch Ermitteln des zeitlichen Verlaufs der Mengen einer durch die Membran (25) hindurch diffundierenden Substanz kann der Diffusions-Koeffizient und/oder die Permeabilität der Membran (25) sowie auch der auf dieser vorhandenen Zellkultur ermittelt werden. Durch geeignete Auswahl der Zellen können Bedingungen geschaffen werden, die denjenigen bei der Resorption der betreffenden Substanz durch die Biophase eines Lebewesens sehr nahekommen.

Fig.1

Fig.2

**Beschreibung**

Membran sowie Einrichtung zur Untersuchung der Diffusion einer Substanz, Verfahren zur Herstellung der Membran und Verfahren zum Betrieb der Einrichtung

Die Erfindung betrifft eine Membran gemäss dem Oberbegriff des Anspruchs 1.

Die Wirkstoffe oral verabreichter Arzneimittel werden üblicherweise durch die Magen und/oder Darmschleimhaut hindurch von der Biophase des betreffenden Menschen oder Tieres resorbiert oder absorbiert. Diese Resorption oder Absorption erfolgt mindestens teilweise durch Diffusion. Bei der Entwicklung und Untersuchung von Arzneimitteln besteht ein grosses Interesse an Kenntnissen über den Ablauf des Resorptions- oder Absorptionsvorgangs. Um möglichst wenig Versuche am lebenden Tier oder Menschen durchführen zu müssen, ist man bestrebt, die Resorption oder Absorption ausserhalb von Körpern lebender Tiere oder Menschen, d.h. in vitro zu simulieren. Ähnliche Probleme stellen sich auch, wenn zum Beispiel das Resorptionsverhalten oder Absorptionsverhalten der einen Körper eines Menschen oder Tieres aussen bedeckenden Haut für in Salben enthaltenen Arzneimittel-Wirksubstanzen oder Bestandteile kosmetischer Produkte untersucht werden soll. Ferner sind Kenntnisse über das Resorptions- oder Absorptionsverhalten auch bei Pflanzen von Nutzen, die zum Beispiel mit Pflanzenschutz- oder Düngemitteln behandelt werden und in diesen vorhandene Substanzen resorbieren oder absorbieren.

Aus der Publikation "Theoretical and Experimental Studies of Transport of Micelle-Solubilized Solutes" von G.E. Amidon, W.I.Higuchi, N.F.H. Ho, in Journal of Pharmaceutical Sciences, Vol. 71, 1982, Seite 77 ist ein Verfahren bekannt, für dessen Durchführung eine Einrichtung mit einem um eine vertikale Achse drehbaren Rotor mit einem hülsenförmigen Wandteil verwendet wird. An dessen unterem, eine Öffnung aufweisenden Ende können mit einer auf ihn aufgeschraubten Klemmhülse zwei Distanzringe, eine gelochte Stützplatte und eine aus Dimethylpolysiloxan bestehende, lipidartige Membran festgeklemmt werden, so dass die letztere zusammen mit dem Wandteil ein Kompartiment begrenzt und dieses gegen ein anderes Kompartiment abgrenzt. Da bei den Untersuchungen mit dieser Einrichtung Membranen mit stark von den Eigenschaften natürlicher Magen- und Darmschleimhäute abweichenden Eigenschaften benutzt werden, sind die sich bei diesen Untersuchungen ergebenden Resultate nur schlecht auf die tatsächlichen Resorptionsvorgänge in Lebewesen übertragbar. Bei lipidartigen Membranen besteht insbesondere bei der Untersuchung gut fettlöslicher Substanzen die Gefahr, dass sich die Substanzen primär in den Membranen anreichern, so dass diese lebende Biomembranen nur schlecht simulieren, da die Substanzen bei echten, d.h. lebenden Biomembranen chemisch verändert, d.h. metabolisiert werden können. Ferner kann es sich störend auswirken, dass der die Diffusion einer Substanz ermöglichende Bereich der Membranen durch die zwischen den Löchern der Stützplatte vorhandenen Stege in relativ kleine, kreisförmige Teilbereiche unterteilt ist. Zudem ist es bei dieser Einrichtung relativ mühsam, eine Membran nach vorübergehendem Losschrauben der Klemmhülse zwischen der Stützplatte und einem der Distanzringe durch Wiederaufschrauben der Klemmhülse zu befestigen und am Ende einer Untersuchung wieder vom Wandteil zu trennen.

Der Erfindung liegt nun die Aufgabe zugrunde, eine Membran zu schaffen, die Nachteile der bekannten Membranen möglichst weitgehend vermeidet und insbesondere ermöglicht, bei den in vitro durchgeführten Untersuchungen Bedingungen zu schaffen, die denjenigen bei Resorptionsvorgängen in oder an Lebewesen, d.h. lebenden Organismen, wie Menschen, Tieren und eventuell Pflanzen möglichst nahe kommen.

Diese Aufgabe wird durch eine Membran der einleitend genannten Art gelöst, wobei die Membran erfindungsgemäss durch den kennzeichnenden Teil des Anspruchs 1 gekennzeichnet ist.

Vorteilhafte Ausgestaltungen der Membran ergeben sich aus den vom Anspruch 1 abhängigen Ansprüchen.

Die Erfindung betrifft ferner ein Verfahren zur Herstellung einer Membran, welches Verfahren erfindungsgemäss durch den kennzeichnenden Teil des Anspruchs 6 gekennzeichnet ist.

Die Erfindung betrifft zudem eine Einrichtung gemäss dem Oberbegriff des Anspruchs 7. Die Einrichtung ist erfindungsgemäss durch den kennzeichnenden Teil dieses Anspruchs gekennzeichnet. Eine günstige Weiterbildung der Einrichtung geht aus dem von diesem Anspruch abhängigen Anspruch hervor.

Die Erfindung betrifft des weitern ein Verfahren zum Betrieb der Einrichtung gemäss dem Oberbegriff des Anspruchs 9, wobei das Verfahren erfindungsgemäss durch den kennzeichnenden Teil dieses Anspruchs gekennzeichnet ist. Eine bevorzugte Ausgestaltung des Verfahrens ergibt sich aus dem von diesem Anspruch abhängigen Anspruch.

Die Membran kann beispielsweise einen flächenhaften, flexiblen, aus einem leblosen, biokompatiblen, vorzugsweise hydrophilen Material bestehenden Träger und von diesem getragene, von mindestens einem Lebewesen abstammende und/oder gebildete, vorzugsweise mindestens zum Teil lebende Zellen aufweisen. Wenn eine Membran zum Beispiel zur Simulation der Resorption einer Substanz durch eine semipermeable Magen- oder Darmschleimhaut eines Lebewesens hindurch vorgesehen ist, hat die Membran als Ganzes vorzugsweise eine möglichst ähnliche Permeabilität oder Semipermeabilität wie die zu simulierende Schleimhaut. Wenn eine Membran aus einem Träger und von diesem getragenen Zellen besteht, setzt sich der der Diffusion einer Substanz entgegenstehende Widerstand aus dem Widerstand des Trägers und dem Widerstand der Zellen zusammen. Der Träger muss also für Flüssigkeiten zumindest semipermeabel, d.h. zumindest für Wasser und/oder eine allfällige andere, als Lösungsmittel dienende Flüssigkeit durchlässig sein und auch den Durchtritt von in gelöster Form vorliegenden Molekülen der zu untersuchenden Substanz ermöglichen, während er grössere Moleküle eventuell zurückhalten kann. Man kann jedoch auch einen Träger vorsehen, der voll permeabel ist, d.h. in einer Flüssigkeit gelöste oder eventuell suspendierte Moleküle beliebiger Grösse durchlässt.

Der Träger kann zum Beispiel aus einer flexiblen, porösen und kompakten, d.h. abgesehen von den Poren zusammenhängenden Folie bestehen. Die Poren bilden mindestens zum Teil den Träger durchdringende Durchgänge, so dass dieser semipermeabel oder eventuell voll permeabel ist. Der Träger kann jedoch auch aus einem flexiblen, flächenhaften Teilstück, wie einem Gewebe, einer Maschenware, einem Geflecht oder einem faserhaltigen Verbundmaterial, d.h. einem Filz oder Vliesstoff bestehen. Falls der Träger aus einem flächenhaften Textilstück besteht, besitzt dieses zwischen benachbarten Fasern und oder Fadenstücken sowie eventuell im Fasermaterial selbst Poren und/oder Öffnungen, die zumindest zum Teil den Träger durchdringende Durchgänge bilden und zum Beispiel so gross sind, dass der Träger nicht nur semipermeabel, sondern voll permeabel ist.

Der Träger kann aus einem synthetischen und/ oder natürlichen Trägermaterial gebildet sein. Geeignete Trägermaterialien sind zum Beispiel Polyacrylnitril, ein Polyacrylnitril-Copolymerisat, Cellulose, Celluloseacetat, Polycarbonat, ein Polycarbonat-Copolymersiat, Baumwolle oder ein für die Bildung von Nähten bei chirurgischen Operationen und Wundbehandlungen verwendbares, sich im Körper eines menschlichen oder tierischen Lebewesens auflösendes oder nicht auflösendes Monofil- oder Multifil-Nahtmaterial. Sich nicht auflösende, bekannte synthetische Nahtmaterialien bestehen zum Beispiel mindestens zum Teil aus Polyamid. Für die Verwendung bei chirurgischen Operationen bestimmte, sich im Körper eines menschlichen oder tierischen Lebewesens auflösende und vom Körper resorbierbare Nahtmaterialien können natürlichen Ursprungs sein, wie etwa Katgut, oder synthetisch hergestellt sein und etwa ganz oder teilweise aus einem Lactat bestehen. Sich auflösende und absorbierbare, synthetische Nahtmaterialien sind zum Beispiel unter den Bezeichnungen Poly-p-dioxanon oder beschichtetes Vicryl (Polyglactin 910) von der Firma Ethicon des Konzerns Johnson & Johnson erhältlich. Von den angegebenen Trägermaterialien sind zumindest Polyacrylnitril und Copolymerisate von diesem lichtdurchlässig oder sogar durchsichtig, was besonders vorteilhaft ist.

Die Träger können zum Beispiel aus im Handel erhältlichen, an sich für die Verwendung bei Hämodialysen vorgesehenen, semipermeablen Folien hergestellt werden. Solche Folien aus Polyacrylnitril oder einem Copolymerisat von diesem sind zum Beispiel von der Firma Rhône-Poulenc, Paris, Frankreich erhältlich. Polycarbonat-, oder Polycarbonat-Copolymerisat-Folien für die Hämodialyse werden von der C. R. Bard Company, Covine, Kalifornien, USA, hergestellt.

Der Träger kann je nach der Art des zu simulierenden Resorptionsvorgangs mit verschiedenartigen, zumindest ursprünglich von mindestens einem Lebewesen abstammenden und/oder gebildeten Zellen versehen werden. Wenn beispielsweise die Resorption einer Arzneimittel-Wirksubstanz durch die Wandungen des Magen- Darmkanals von Menschen simuliert werden soll, können auf dem Träger für die Herstellung der Membran Intestinalzellen kultiviert werden. Solche Zellen, die humanen Ursprungs sind, können bei Zellbanken bezogen werden, wobei zum Beispiel Versuche mit gemäss internationalen Konventionen als "Intestine" Nr. 407 bezeichneten Zellen durchgeführt wurden. Wenn hingegen die Resorption einer in einer Salbe oder Crème vorhandenen Arzneimittel-Wirksubstanz oder einer Substanz eines kosmetischen Produkts durch die Aussenhaut, d.h. die äussere Bedeckung eines menschlichen Körpers simuliert werden soll, können auf dem Träger Haut-Zellen, Muskelzellen oder Fibroblasten der Vorhaut humanen Ursprungs kultiviert werden. Für die Simulation von in oder an menschlichen und tierischen Lebewesen stattfinden Resorptionsvorgängen können selbstverständlich auch tierische Zellen auf dem Träger kultiviert werden. Falls die Resorption von zur Behandlung von Pflanzen dienenden Substanzen, beispielsweise Bestandteilen von Pflanzenschutz- oder Düngemitteln oder sonstigen Agrochemikalien, durch Pflanzen simuliert werden soll, können auf den Trägern auch Zellen pflanzlichen Ursprungs kultiviert werden. Des weitern besteht die Möglichkeit, ein- oder mehrzellige Klein-Lebewesen, wie zum Beispiel Algen oder Pilze, auf den Trägern zu kultivieren.

Die für die Diffusionsuntersuchungen vorgesehene Einrichtung weist vorzugsweise einen formfesten, mit etwa bajonettverschlussartigen Schnellverschluss-Verbindungsmitteln lösbar an einen Rotor der Einrichtung befestigten, ring- und/oder hülsenförmigen, eine Achse umschliessenden Wandteil auf, der dann beim Durchführen einer Untersuchung zusammen mit der Membran ein Donator-Kompartiment begrenzt. Die Festigkeit der zumindest normalerweise flexiblen Membran und die Ausbildung des Wandteils sollen vorzugsweise derart aufeinander abgestimmt sein, dass der mittlere sich über die Öffnung des Wandteils erstreckende Abschnitt der Membran selbsttragend ist. Die Membran soll also mit anderen Worten gesagt stark genug sein, dass ein entlang des Umfangs des Wandteils an diesem befestigter Randabschnitt mit der Membran deren das Hindurchdiffundieren einer Substanz ermöglichenden, unterbruchslos zusammenhängenden, mittleren Abschnitt trägt, ohne dass dieser zusätzlich mit einer starren, perforierten Stützplatte abgestützt werden muss.

Der Träger kann zum Besiedeln mit den genannten Zellen oder Lebewesen lösbar am von der restlichen Einrichtung getrennten Wandteil befestigt werden. Danach können Zellen oder Klein-Lebewesen auf den Träger aufgeimpft und beispielsweise in einem sterilen Brutschrank kultiviert werden.

Da das Kultivieren von Zellen oder Kleinlebewesen im allgemeinen wesentlich länger dauert als die Durchführung einer Untersuchung des Diffusionsverhaltens mit Bestimmung des Diffusionskoeffizienten und/oder der Permeabilität der Membran, kann eine Einrichtung mit mehreren lösbar am Rotor befestigbaren Wandteilen der genannten Art ausgerüstet werden, so dass gleichzeitig oder während einander überlappenden Zeiträumen auf mehreren an je einem Wandteil befestigten Trägern Zellen kultiviert werden können.

Durch Kultivieren von Zellen auf einem Träger kann man wahlweise Membranen mit nur einer einzigen Schicht von miteinander verwachsenen Zellen, einem sogenannten Zellrasen, oder mit mehreren übereinander gewachsenen Schichten von Zellen, einem Gewebe bilden. Die Zellkulturen können während des Kultivierungsvorgangs und vor der Durchführung einer Untersuchung des Diffusionsverhaltens jeweils mikroskopisch untersucht werden. Dadurch lässt sich sicherstellen, dass die Kultur die gewünschte Beschaffenheit hat und beispielsweise im Fall eines Zellrasens eben tatsächlich den ganzen freien. d.h. eine Diffusion ermöglichenden Bereich des Trägers mindestens einigermassen und vorzugsweise vollkommen lükkenlos bedeckt. Wenn die Träger, wie zum Beispiel Träger aus Polyacrylnitril und Copolymerisaten von diesem, lichtdurchlässig oder durchsichtig sind, können die mikroskopischen Begutachtungen mit Durchlicht, d.h. mit den betreffenden Träger durchdringendem Licht vorgenommen werden. Dies ermöglicht zum Beispiel die Verwendung eines Umkehrmikroskops, was die Beurteilung der Zellkultur erleichtert oder verbessert. Im übrigen können die Zellen selbstverständlich auch nach der Untersuchung des Diffusionsvorgangs mikroskopisch untersucht werden. Falls auf den Trägern Klein-Lebewesen kultiviert werden, können selbstverständlich auch diese Kulturen, analog wie es vorgängig für die Zellkulturen erwähnt wurden, mikroskopisch untersucht werden.

Wenn ein an einem Wandteil befestigter Träger in vom erwähnten Rotor getrennten Zustand mit einer Kultur von Zellen oder solche aufweisenden Klein-Lebewesen versehen wurde, kann der Wandteil mitsamt der vorgängig mittels eines Befestigungselementes an ihm befestigten, lebende Zellen aufweisenden Membran lösbar am Rotor befestigt werden. Dieser Befestigungsvorgang kann in einer Weise durchgeführt werden, bei der zumindest der wesentliche Teil der Membran, nämlich insbesondere deren mit Zellen besiedelter Abschnitt und vorzugsweise die ganze am Wandteil befestigte Membran mit keinem weiteren, festen Teil der Einrichtung in Berührung gelangt. Dementsprechend wird die Membran durch die Befestigung am Rotor keinerlei mechanischen Beanspruchungen unterworfen, was gewährleistet, dass die auf dem Träger der Membran vorhandenen Zellen trotz ihrer Empfindlichkeit auf mechanische Beanspruchungen weder vom Träger abgelöst noch sonst irgendwie geschädigt werden.

Die Membranen können anstelle von Kulturen von Zellen oder Klein-Lebewesen auch Zellen oder Gewebeteile aufweisen, die als Ganzes lebenden Tieren oder Menschen oder tierischen oder menschlichen Leichen entnommen werden. Die Membranen können beispielsweise Stücke der Magen- oder Darmwandungen oder mindestens der Schleimhaut dieser Wandungen oder der Aussenhaut eines Menschen oder Tieres enthalten. Falls diese vorzugsweise lebenden oder eventuell toten Haut- oder sonstigen Gewebeteile eine ausreichende Festigkeit haben und keine Löcher aufweisen, können sie freitragend am erwähnten Wandteil befestigt werden. Andernfalls können sie ebenfalls auf einem Träger angeordnet und in irgend einer Weise an diesem befestigt werden.

Die Substanz, deren Diffusionsverhalten mittels einer Membran untersucht wird, kann aus einem einzigen chemischen Stoff, d.h. einem chemischen Element oder einer Verbindung, bestehen oder durch ein Gemisch mit zwei oder mehr Komponenten gebildet sein. Die erfindungsgemässen Membranen können insbesondere zur Untersuchung der Resorption oder Absorption von Azneimittel-Wirksubstanzen und/oder -Hilfssubstanzen verwendet werden. Solche Substanzen sind zum Beispiel Salicylsäure, Ascorbinsäure, Urea, Zucker, Aminosäuren, Peptide, Proteine, Calcitonin, Cyclosporin, Interleukin, Progesteron und Interferon. Es können also sowohl Substanzen mit verhältnismässig kleinen Molekülen, wie die als Bausteine dienenden Aminosäuren, als auch verhältnismässig hochmolekulare Substanzen, wie Cyclosporin, Calcitonin, Interleukin, Interferon usw. untersucht werden. Falls die Tests mit je einen Träger aufweisenden Membranen durchgeführt werden, können die Träger nötigenfalls derart ausgewählt oder hergestellt werden, dass ihre von Poren oder Öffnungen gebildeten Durchgänge abhängig von den Molekülgrössen der zu untersuchenden Substanzen kleinere oder grössere Querschnittsabmessungen haben.

Bei der Durchführung einer Untersuchung eines Diffusionsvorgangs kann durch eine Membran ein Donator-Kompartiment von einem Akzeptor-Kompartiment abgegrenzt werden. Die zu untersuchende Substanz kann in einem wässrigen und/oder möglicherweise anderen flüssigen oder pastösen Lösungsmittel zumindest zum Teil gelöst und/oder in einer Flüssigkeit dispergiert oder eventuell auch in Liposomen gebunden oder in Arzneimittel-Kapseln in das Donator-Kompartiment eingebracht werden. Falls die Resorption einer in einer Salbe oder Crème enthaltenden Substanz simuliert werden soll, kann man diese halbfeste Salbe oder Crème in das Donator-Kompartiment einbringen. Beim Hindurchdiffundieren durch die Membran ist die Substanz dann jedoch in jedem Fall in einem Lösungsmittel gelöst.

Die zu untersuchende Substanz wird vorzugsweise mit einem radioaktiven Isotop, wie $C^{14}$ oder Tritium, markiert. Das Akzeptor-Kompartiment kann eine vorzugsweise wässerige Flüssigkeit enthalten. Falls zum Beispiel die Resorption im Magen-Darmkanal simuliert werden soll, kann im Donator-Kompartiment eine Pufferlösung vorhanden sein, deren pH-Wert demjenigen der Verdauungssäfte im Magen bzw. Darm entspricht. Die im Akzeptor-Kompartiment vorhandene Akzeptor-Flüssigkeit wird fortlaufend über eine Messvorrichtung umgewälzt, die einen Detektor zum Nachweis radioaktiver Strahlen, etwa einen Szintillationszähler aufweist. Man kann dann laufend die Menge oder Konzentration der in der umgewälzten Flüssigkeit vorhandenen, durch die Membran hindurchdiffundierten Substanz ermitteln und aus der Zunahme der Substanzmenge in bekannter Weise für die betreffende Substanz den Diffusionskoeffizienten D und/oder den Permeabilitätskoeffizienten P ermitteln. Der letztere ist mit dem Diffusionskoeffizienten D verknüpft. Die Verknüpfung kann dargestellt werden durch die Formel

$$P = D \bullet K/h$$

Dabei ist h die Dicke der Membran und K der Verteilungkoeffizient. Der letztere ist gleich dem Quotienten Sättigungslöslichkeit der untersuchten Substanz in der Membran durch die Sättigungslöslichkeit der Substanz im Lösungsmittel. Die Substanzmengen können bei entsprechender Beschaffenheit oder Vorbehandlung der Substanzen statt durch Radioaktivitätsmessungen auch durch Fluoreszenzmessungen oder spektrophotometrisch ermittelt werden. Falls die zu untersuchende Substanz im Donator-Kompartiment als Bestandteil einer Lösung vorhanden ist, könnte man zur Ermittlung der Menge der durch die Membran hindurch diffundierten Substanz statt der Zunahme der Substanzmenge in der durch das Akzeptor-Kompartiment umgewälzten Flüssigkeit oder zusätzlich dazu die Abnahme der Substanzmenge im Donator-Kompartiment messen.

Wenn die im Akzeptor-Kompartiment vorhandene Flüssigkeit bezüglich der Membran ruhend ist, entsteht im Akzeptor-Kompartiment in der Nähe der Membran eine ruhende Grenzschicht, wodurch die Diffusion gehemmt wird. Das Donator-Kompartiment wird nun beim Messen des Diffusionskoeffizienten und/oder Permeabilitätskoeffizienten vorzugsweise derart um eine vertikale Achse gedreht, dass sich die Membran bezüglich des Akzeptor-Kompartiments dreht und in diesem im an die Membran angrenzenden Bereich eine laminare Strömung entsteht. Die genannte Grenzschicht wird durch die Strömung auf eine verhältnismässig dünne, bezüglich der Membran mehr oder weniger ruhende Grenzschicht reduziert, wobei die Dicke dieser Grenzschicht mit wachsender Drehzahl abnimmt. Man kann nun zum Beispiel die Drehzahl des Donator-Kompartiments variieren, für verschiedene Drehzahlen die Permeabilität ermitteln und durch eine Extrapolation der Messwerte die einer unendlich grossen Drehzahl entsprechende Permeabilitätskoeffizienten bestimmen. Dieser ist dann gleich dem Wert des Permeabilitätskoeffizienten, den die Membran bei einer Grenzschicht mit der Dicke Null hätte. Die gegebenenfalls im Donator-Kompartiment vorhandene Flüssigkeit wird beim Drehen des Donator-Kompartiments zwar mehr oder weniger von diesem mitbewegt, wobei sich aber insbesondere im Fall, dass die Drehzahl sukzessive gesteigert wird, ebenfalls noch eine Strömung ausbildet, welche die sich im Donator-Kompartiment ergebende, ein Substanz-Konzentrationsgefälle aufweisende Grenzschicht dünner macht. Abgesehen davon, dass diese Grenzschicht weniger stört als die sich im Akzeptator-Kompartiment ergebende Grenzschicht, kann man nötigenfalls mindestens eine bezüglich des Akzeptor-Kompartiments feststehende, ins Donator-Kompartiment hineinragende Schikane vorsehen, die die im Donator-Kompartiment vorhandene Flüssigkeit beim Drehen des letzteren bremst, so dass die Grenzschicht im Donator-Kompartiment durch Drehen des letzteren ebenfalls auf eine geringe Dicke reduziert werden kann.

Wenn bei der Untersuchung des Diffusionsverhaltens einer bestimmten Substanz eine Membran verwendet wird, die aus einem Träger und einer von diesem getragenen Zellkultur besteht, kann zusätzlich zum Permeabilitätskoeffizienten dieser Membran auch noch der Permeabilitätskoeffizient ermittelt werden, der sich für die betreffende Substanz ergibt, wenn der Träger allein als Membran dient. Man kann danach die Differenz der Reziprokwerte der beiden Permeabilitätskoeffizienten berechnen. Der Reziprokwert der Differenz gibt dann den Permeabilitätskoeffizienten des aus den Zellen bestehenden Teils der Membran an. Bei geeigneter Auswahl der Zellen liegt dieser Wert relativ nahe beim Permeabilitätskoeffizienten, der sich in vivo bei einem Lebewesen ergibt oder ist zumindest in sehr guter Näherung proportional zum letztgenannten Permeabilitätskoeffizienten.

Mit der nachfolgend noch näher beschriebenen Einrichtung ist zum Beispiel die Resorption von Salicylsäure, Ascorbinsäure, Urea und dem Hormon Progesteron untersucht worden. Ein Vergleich der ermittelten Permeabilitätskoeffizienten mit in der Literatur publizierten, mit in vivo Verfahren an Rattendünndärmen gemessenen Werten ergab insbesondere im Fall von Salicylsäure und Progesteron eine verhältnismässig gute Übereinstimmung.

Die erzielbare, weitgehende Übereinstimmung zwischen mittels der Einrichtung durchgeführten in vitro Messungen und der in vivo ablaufenden Resorptionsvorgänge ermöglicht also in gewissen Fällen, weitgehend auf in vivo Untersuchungen, zum Beispiel Tierversuche zu verzichten. In anderen Fällen sind jedoch Vergleichsuntersuchungen an Lebewesen nicht zu vermeiden, um zum Beispiel nötigenfalls einen Umrechnungsfaktor zu bestimmen, mit dem die mittels der Einrichtung bestimmten Diffusionskoeffizienten und/oder Permeabilitätskoeffizienten auf in vivo Verhältnisse beim Menschen oder Tier umgerechnet werden können. Auch in diesen Fällen ermöglicht jedoch die Verwendung erfindungsgemässer Membranen sowie der erfindungsgemässen Einrichtung zumindest eine beträchtliche Reduktion von in vivo Versuchen.

Die Erfindung wird nun anhand eines in der Zeichnung dargestellten Ausführungsbeispiels erläutert. In der Zeichnung zeigt

die Figur 1 einen schematisierten Schnitt einer Einrichtung zur Untersuchung des Diffusionsverhaltens sowie zur Ermittlung des Diffusionskoeffizienten und/oder der Permeabilitätskoeffizienten und

die Figur 2 einen Schnitt durch einen Abschnitt einer Membran, in grösserem Massstab.

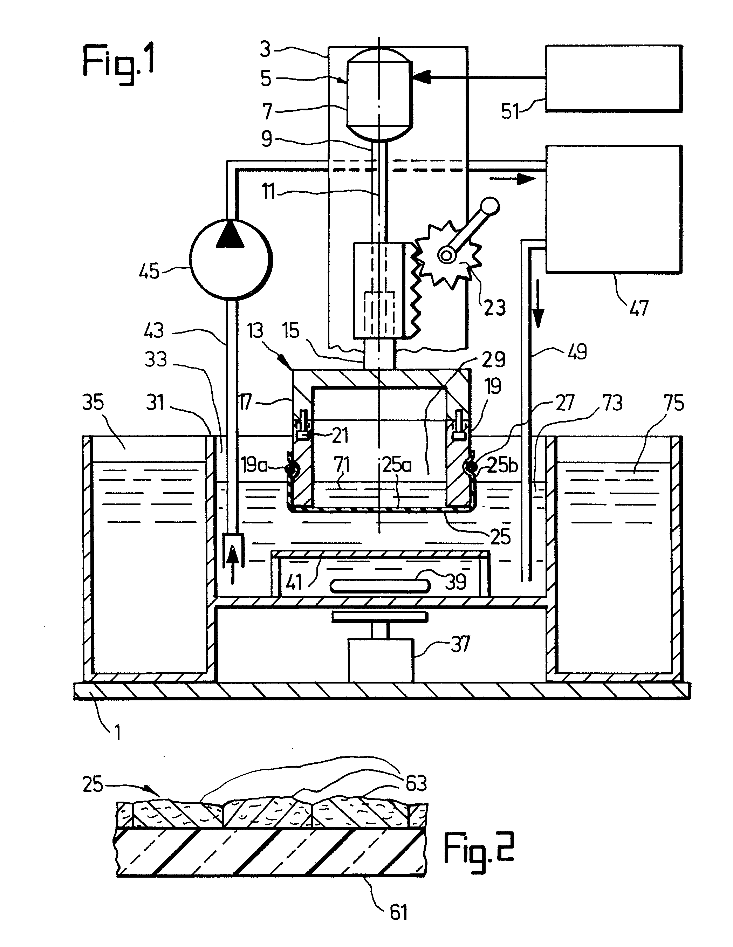

Die in der Figur 1 ersichtliche Einrichtung weist einen Support 1 mit einem Ständer 3 auf, der eine Antriebsvorrichtung 5 mit einem Elektromotor 7 und einer um eine vertikale Achse 11 drehbaren Welle 9 besitzt. Ein Rotor 13 besitzt eine drehfest, aber beispielsweise axial verschiebbar mit der Welle 9 verbundene, zum Beispiel hohle Welle 15 und einen starr mit dieser verbundenen Halteteil 17, der etwa kappenartig ausgebildet ist und oben und/oder seitlich mindestens eine zum Beispiel mit irgend einem Verschlusselement verschliessbare Öffnung besitzt. Am Halteteil 17 ist ein hülsenförmiger, hohlzylindrischer, beidenends offener, zum Beispiel aus eloxiertem Aluminium bestehender Wandteil 19 durch Schnellverschluss-Verbindungsmittel 21 lösbar befestigt. Die letzteren sind beispielsweise in der Art von bajonettverschlussartigen Verbindungsmitteln ausgebildet und weisen an einem der miteinander zu verbindenden Teile, etwa dem Halteteil 17 befestigte Nocken oder Zapfen mit einem Hals oder Schaft und einem radial über diesen hinausragenden Kopf auf und können mit dem letzteren in eine Nut oder einen Schlitz des andern Teils eingreifen. Die zylindrischen Aussenfläche des Wandteils 19 ist unterhalb der Verbindungsmittel 21 mit einer Ringnut 19a versehen. Der Rotor 13 oder mindestens dessen Halteteil 17 kann mittels einer beispielsweise manuell betätigbaren Verstellvorrichtung entlang der Achse 11 ver schoben, d.h. gehoben und abgesenkt werden, wobei die untere, in der Figur 1 dargestellte Endstellung durch Anschlagsmittel festgelegt ist. Statt nur den Rotor 13 verstellbar auszubilden, könnte man auch vorsehen, den Rotor zusammen mit der ganzen Antriebsvorrichtung vertikal zu verstellen. Eine flexible Membran 25 hat einen in axialer Projektion innerhalb der Innenfläche des Wandteils 19 liegenden, unterbruchslos freien, d.h. keinen festen Teil berührenden, mittleren Abschnitt 25a, der bei der unteren, ringförmigen Endfläche des Wandteils 19 einen ebenen Kreis bildet, und einen an der genannten Wandteil-Endfläche sowie an der Aussenfläche des Wandteils 19 anliegenden Randabschnitt 25b. Dieser ist mit einem Befestigungselement 27 lösbar am Wandteil 19 befestigt, wobei das Befestigungselement aus einem gummielastischen O-Ring besteht, der einen Teil des Randabschnitts 25b in die Ringnut 19a hineindrückt. Der Wandteil 19 begrenzt zusammen mit der Membran 25 ein erstes Kompartiment, nämlich das Donator-Kompartiment 29.

Ein zum Beispiel auf dem Support 1 stehender und eventuell an diesem befestigter Behälter 31 begrenzt ein zweites, oben offenes Kompartiment, des Akzeptor-Kompartiment 33. Wenn sich das Donator-Kompartiment 29 in der gezeichneten, unteren Endstellung befindet, ragt es in das Akzeptor-Kompartiment 33 hinein. Das letztere ist von einer ringförmigen Kammer 35 umschlossen. Unter dem Boden des Akzeptor-Kompartiments befindet sich eine Antriebsvorrichtung 37 mit einer Magnetkupplung, die durch den genannten Boden hindurch in magnetischer Dreh-Wirkverbindung mit einem stäbchenförmigen Rührorgan 39 steht. Zwischen diesem und der Membran 25 ist ein scheibenförmiges Abdeckelement 41 angeordnet.

Das Akzeptor-Kompartiment 33 ist durch eine Leitung 43 über einer Saugpumpe 45 mit dem Eingang einer Messvorrichtung 47 verbunden, von deren Ausgang eine Leitung 49 ins Akzeptor-Kompartiment zurückführt. Die Messvor richtung enthält einen Szintillationszähler und einen beispielsweise digitalen Anzeigeteil und/oder eine Registriervorrichtung oder einen Drucker. Der Motor 7 ist elektrisch mit einer Steuervorrichtung 51 zum Einstellen der Drehzahl verbunden.

Die separat in der Figur 2 dargestellte Membran 25 weist einen flexiblen Träger 61 auf, der aus einer porösen, semipermeablen Folie aus einem leblosen, biokompatiblen, hydrophilen, lichtdurchlässigen Material, etwa einem Polyacrylnitril-Copolymerisat besteht und beispielsweise eine im Bereich von 0,01 bis 0,03 mm liegende Dicke hat. Der Träger wird bei der Vorbereitung einer Diffusions-Untersuchung am vom restlichen Rotor getrennten Wandteil 19 befestigt, und auf der obern Seite seines die Diffusion ermöglichenden Bereichs, d.h. seines mittleren Abschnitts 25a mit Zellen 63 beimpft. Diese werden dann in einem Brutschrank kultiviert, so dass sie beispielsweise einen den ganzen Mittelabschnitt 25a bedeckenden einschichtigen Zellrasen bilden. Der Halteteil 17 wird mittels der Verstellvorrichtung 21 vorübergehend soweit nach oben gehoben, dass das Donator-Kompartiment bequem am Halteteil befestigt werden kann. Wenn das Donator-Kompartiment mittels der Verbindungsmittel 21 am Halteteil 17 befestigt, ist kann es mitsamt diesem in die in der Figur 1 gezeichnete Endstellung abgesenkt werden. Danach wird durch die bzw. eine Öffnung des Halteteils 17 hindurch eine Donator-Flüssigkeit 71 mit der zu untersuchenden Substanz, zum Beispiel mit einem radioaktiven Isotop markiertem Harnstoff, in das Donator-Kompartiment 29 eingebracht. Ferner wird in das Akzeptor-Komptartiment eine Abkzeptor-Flüssigkeit 73 eingebracht. Der Halteteil 17 kann nach dem Einfüllen der Donator-Flüssigkeit in einen Zustand gebracht werden, in der er das Donator-Kompartiment oben zur Vermeidung der Verdampfung und/oder Verdunstung von Donator-Flüssigkeit zumindest einigermassen oder vollkommen gasdicht abschliesst. Stattdessen oder zusätzlich könnte auch eine sowohl das Akzeptor- als auch das Donator-Kompartiment ab deckende, entfernbare Haube vorgesehen werden. Die Kammer enthält eine Flüssigkeit 75.

Beim Untersuchen des Diffusionsverhaltens wird die Flüssigkeit 75 mit einer nicht dargestellten Regelvorrichtung auf einer vorgegebenen, zum Beispiel 37°C betragenden Temperatur gehalten, wodurch auch die im AkzeptorKompartiment enthaltene Flüssigkeit 73 auf diese Temperatur gebracht wird. Das Donator-Kompartiment, das mit dem mittleren Abschnitt 25a der Membran in die Flüssigkeit 73 eingetaucht ist, wird mit der Antriebsvorrichtung 5 um die Achse 11 gedreht. Ferner wird auch das Rührorgan 39 mit der Antriebsvorrichtung 37 gedreht, so dass die Akzeptor-Flüssigkeit 73 möglichst gut durchmischt wird, wobei das Abdeckelement 41 verhindert, dass im Bereich der Membran 25 durch Sogwirkung eine Trombe entsteht. Die Akzeptor-Flüssigkeit 73 wird mittels der Pumpe 45 durch die Messvorrichtung hindurch umgewälzt. Die letztere misst die Menge bzw. Konzentration der in der Akzeptor-Flüssigkeit vorhandenen, radioaktiv markierten, durch die Membran 25 hindurch vom Donator-Kompartiment in das Akzeptor-Kompartiment diffundierten Substanz.

**Patentansprüche**

1. Membran zur Untersuchung der Diffusion einer Substanz durch die Membran hindurch, insbesondere um die Resorption einer Arzneimittel-Wirksubstanz und/oder -Hilfssubstanz, einer kosmetischen Substanz oder einer Substanz für die Pflanzenbehandlung durch ein Lebewesen zu simulieren, dadurch gekennzeichnet, dass die Membran mindestens zum Teil aus Zellen (53) besteht, die von mindestens einem Lebewesen abstammen und/oder gebildet sind.

2. Membran nach Anspruch 1, dadurch gekennzeichnet, dass sie einen flächenhaften, für Flüssigkeiten zumindest semipermeablen, aus einem aus leblosen Material bestehenden, vorzugsweise hydrophilen Träger (61) und von diesem getragene, vorzugsweise zumindest zum Teil lebende Zellen (63) aufweist, wobei der Träger (61) zum Beispiel Polyacrylnitril, ein Polyacrylnitril-Copolymerisat, Cellulose, Celluloseacetat, Polycarbonat ein Polycarbonat-Copolymerisat, Baumwolle oder Nahtmaterial für chirurgische Operationen und/oder zum Nähen von Wunden aufweist und zum Beispiel durch eine poröse Folie oder ein flächenhaftes Textilstück gebildet ist.

3. Membran nach Anspruch 2, dadurch gekennzeichnet, dass vom Träger (61) getragene Zellen (53) einen Zellrasen, der zum Beispiel Intestinalzellen, Fibroblasten, Muskelzellen oder Hautzellen aufweist, und/oder ein- und/oder mehrzellige Lebewesen, wie Algen und/oder Pilze, bilden.

4. Membran nach Anspruch 2 oder 3, dadurch gekennzeichnet, dass der Träger (61) lichtdurchlässig und beispielsweise durchsichtig ist.

5. Membran nach Anspruch 1, 2 oder 4, dadurch gekennzeichnet, dass sie mindestens zum Teil aus einem mehrere Zellschichten aufweisenden, vorzugsweise zumindest zum Teil lebende Zellen besitzenden oder eventuell abgestorbenen Gewebe besteht, wobei das Gewebe zum Beispiel ein Stück der Aussenhaut oder der Magen- oder Darmschleimhaut eines Menschen oder Tieres oder der äussern Begrenzungsschicht einer Pflanze aufweist.

6. Verfahren zur Herstellung einer Membran nach einem der Ansprüche 1 bis 4, dadurch gekennzeichnet, dass auf einem aus leblosem Material bestehenden Träger (61) Zellen (63) und/oder ein- und/oder mehrzellige Lebewesen kultiviert werden.

7. Einrichtung zur Untersuchung der Diffusion einer Substanz durch eine Membran (25) hindurch, insbesondere einer Membran nach einem der Ansprüche 1 bis 5, beispielsweise um die Resorption einer Arzneimittel-Wirksubstanz und/oder -Hilfssubstanz, einer kosmetischen Substanz oder einer Substanz für die Pflanzenbehandlung durch ein Lebewesen zu simulieren, mit einem durch eine Antriebsvorrichtung (5) um eine vertikale Achse (11) drehbaren Rotor (13), einem drehfest mit diesem verbundenen Wandteil (19), wobei der letztere ausgebildet ist, um zusammen mit der lösbar an ihm befestigten Membran (25) ein erstes Kompartiment (29) zu begrenzen, und einem zweiten Kompartiment (33) zum Aufnehmen einer bei der Untersuchung der Diffusion an die untere Seite von mindestens einem Bereich der Membran (25) angrenzenden Flüssigkeit (73), dadurch gekennzeichnet, dass der Wandteil (19) lösbar mit dem Rotor (13) verbunden und eine Verstellvorrichtung (21) vorhanden ist, um zumindest den lösbar mit dem Wandteil (19) verbundenen Teil (17) des Rotors (15) und das zweite Kompartiment (29) bezüglich einander entlang der Achse (11) zu verstellen.

8. Einrichtung nach Anspruch 7, dadurch gekennzeichnet, dass der Wandteil (19) und zum lösbaren Befestigen der Membran (25) dienende Mittel (27) derart ausgebildet sind, dass derjenige Membran-Bereich, der dazu bestimmt ist, die Substanz hindurchdiffundieren zu lassen, einen einzigen, unterbruchslos zusammenhängenden Abschnitt (25a) bildet, wobei der Wandteil (19) und der Rotor (13) vorzugsweise durch Schnellverschluss-Verbindungsmittel (21), zum Beispiel bajonettverschlussartige Verbindungsmittel, miteinander verbunden sind.

9. Verfahren zum Betrieb der Einrichtung nach Anspruch 7 oder 8, wobei die Membran (25) für die Untersuchung der Diffusion lösbar an einer Stirnseite eines eine Achse (11) umschliessenden Wandteils (19) befestigt wird und dadurch ein von diesem und der Membran (25) begrenztes, erstes Kompartiment (29) gebildet und dieses mit sich unten befindender Membran (25) in Kontakt mit einer sich im zweiten Kompartiment (33) befindenden Flüssigkeit (73) gebracht sowie um die genannte, vertikale Achse (11) gedreht wird, wobei die Menge der von einem Kompartiment (29) durch die Membran (25) hindurch diffundierten Substanz ermittelt wird, dadurch gekennzeichnet, dass der Wandteil (19) in vom Rotor (13) getrenntem Zustand mit der Membran (25) versehen und zusammen mit dieser am Rotor (13) befestigt wird.

10. Verfahren nach Anspruch 9, dadurch gekennzeichnet, dass am vom Rotor (13) getrennten Wandteil (19) zur Bildung der Membran (25) ein aus leblosem, vorzugsweisen hydrophilem Material bestehender Träger (61) befestigt wird und dass der Träger (61) darnach mit einer Kultur von lebenden Zellen (63) und/oder ein- und/oder mehrzelligen Lebewesen besiedelt und die derart gebildete Membran (25) anschliessend gemeinsam mit dem Wandteil (19) am Rotor (13) befestigt wird, wobei bei der Untersuchung der Diffusion vorzugsweise sowohl die Menge der durch den mit Zellen (63) und/oder Lebewesen besiedelten Träger (61) als auch die Menge der durch den zellenlosen bzw. lebewesenlosen Träger (61) hindurch diffundierten Substanz gemessen und vorzugsweise daraus eine Grösse errechnet wird, die das Diffusionsverhalten charakterisiert, das die Zellen (63) bzw. Lebewesen allein für die Substanz ergäben, wobei die genannte, errechnete Grösse zum Beispiel der Permeabilitätskoeffizient ist.

Fig.1

Fig.2

0242326